Europäisches Patentamt

European Patent Office

Office européen des brevets

(11) Publication number: **0 329 834**
**A2**

(12) **EUROPEAN PATENT APPLICATION**

(21) Application number: 88119393.2

(22) Date of filing: 22.11.88

(51) Int. Cl.⁴: **A61K 7/06 , A61K 7/48 , A61K 35/78 , A61K 7/26**

(30) Priority: 28.01.88 US 150771

(43) Date of publication of application:
30.08.89 Bulletin 89/35

(84) Designated Contracting States:
AT BE CH DE ES FR GB GR IT LI LU NL SE

(71) Applicant: Izundegui MacDonnell, Francisco
José
1 de Mayo 222 Colonia Garcia
MX-86040 Villahermosa Tabasco(MX)

(72) Inventor: Izundegui MacDonnell, Francisco
José
1 de Mayo 222 Colonia Garcia
MX-86040 Villahermosa Tabasco(MX)

(74) Representative: Dorner, Jörg, Dr.-Ing.
Dorner & Hufnagel Patentanwälte
Ortnitstrasse 20
D-8000 München 81(DE)

(54) Scalp hair regeneration and regrowthing and skin, mucosas healing and germicide composition.

(57) The invention is concerned with the scalp's hair regrowth property, the scalp' dandruff control property, the skin and mucosas healing and regenerating properties of bruses, excoriations, abrasions, burns and sores and ulcers through the action of the regenerating and healing property of the composition contained as active ingredients in the extracts of the cortex, small branches and leaves from the plant Mimosa Tuitenuiflora Leguminosae from the Southeast part of MEXICO and the ground slightly roasted powder derived from it and ac-tive ingridients contained in the plant.

EP 0 329 834 A2

## Scalp hair regeneration and regrowthing and skin, mucosas healing and germicide composition.

The subject of my invention is a hair growthing composition and skin and mucosas treating and healing composition than can be used for burns, ulcers and abrations successfully.

No other natural remedy has been described before that promotes hair regeneration and regrowth capacity and also promotes skin and mucosas healing and regenerating capacity which is pwrfectly non noxious, safe, with rapid results and effective in treating hair lost in the scalp providing that the hair follicle exists and can be activated back into hair regrowth and also cheking or stoping dandruff formation. In particular the present invention is directed to compositions for the treatment of baldness, dandruff, abrasions, excoriations, burns of first, second and third degrees and ulcers either from the skin or from the mucosas, specially those derived from leg varicose vains complications and ulcers from bed sores and women's ulcers from the cervix of the uterus.

The composition according to the present invention contains an extract or preparation in different forms from the cortex, small branches and leaves of the one year old or older of the Southeast Mexican tree Mimosa Tuitenui flora Leguminosae POIR. It has not been described ever before that the --parts mentioned of this plant to have the propitious faculty of promoting the regrowth of the hair from the scalp and also skin and mucosas healing and regeneration and infection protection.

The present invention can be extracted from the cortex, small branches and leaves of said plant by hot water immersion or throughout the action excerted by the known solvents such as alcoholes, etheres, glyceroles, glycoles acetones, naftas, petroleum derivatives, by chromatografic procedures by -hot mineral, vedgetable and animal oils and fats by dialisis or osmosis by centrifugation. It also can be prepared by simply reducing it to powder -- form by grinding, crushing, cutting in small fragmentsor rolling of the --dried cortex, small branches or leaves from the Mimosa Tuitenuiflora tree and then subjecting it to heating in a rolling circular metalic oven until a -slight brown roasted state is obtained that after autoclave sterelization can be applied on the skin and mucosa lesions. Also can be proceded by extracting only one part separately until the dry substance content is achieved. The obtained extracts can be mixed or used alone upon the requested -concentration.

I have found that only one to three applicationsof the dried and roasted slightly and finely ground powder as thin covering on abrasions, burns, skin and mucosas ulcerations and open wounds even extensive and deep will -cause them to healin a prompt manner and without pain and also controlling localinfection produced by fungus or bacteria. The application of this powder can be achieved solely by putting it inside a container with a perforated cap or medical powder insuflator and shaking it over the external lesions or insuflating it inside body cavities with lesions or ulcer. For stomach and intestinal ulcers can be swallowed in capsules or introduced suspended in water as enemas or cholonic irrigations. Also can be used as a tea. The plant cortex or small branches and dried leaves are preferably chopped and ground in a hammer mill. Extraxtion can be carried out at atmosphere pressureand temperatures not to exceed 80° C. to preserve the organic components of the plant. Any known method can be used, choice depend on the scale of the method. Continuos, semicontinuos or discontinuos methods providing that it does not causes abrupt shaiking or stirring because foam formation in large quantities that will impair the extraction procedure. Should not -produce any longer, extraction can be finished. The solutions are filtered and the one extracted with water can be mixed with water-ethanol 96° proff in the proportion required to make a lotion for scalp application in variable concentrations. Also the extracts obtained by hot water treatment can be vaccum concentrated and lyophyliced to powder and then kept for further use in several mixing procedures.

The solution prepared according to our invention is to be finished and can be directly applied or optionally after addition of certain additives can be directly applied or used as bathing but also can be prepared as spray, cream, soap, shampoo, paste or powder.

The composition according to my invention promotes cell regeneration and normal regrowth on all wounded or burnt or ulcerated skin and mucosas lesions even so they are of long standing nature, large and deep.

The composition according to my invention is to be used as follows:any strange material and crostes should be cleaned with soap and water scrubbing, all dead tissue should be remouved out by means of surgical disection, once the lesion looks and is clean the sterilized fine and slightly roasted powder is applied or insuflated in thin layer on the lesion. No dressing is necessary due to this powder forms a protection crost itself of curative and germicidal action. This application is repeatedtwo or three times every 24 hours. Usually it takes in the majority of the small and medium size lesions this three or four applications to heal up completly.

In oerder to obtain scalp hair regrowth it is necessary that the lotion be be applied every day at bed time preferibly and the next morning not necessarily but convinient to wash the scalp with a soap or shampoo in wich formula also enters the extract or the powder described above.

For small bruises and abrations or burns the oinment composition or cream or also the oily preparation can be applied as household popular medication or at schools or for use in lesions of animals with valuable furs. No espertice is required when it is used for treating simple lesions.

EXAMPLE 1:

One kilogram ofdried raw cortex, small branches or leaves of the one year or older Mimosa Tuitenuiflora Leguminosae three is deeped in water that is heated up to 80° C during 24 hours. Water and matter extracted is -drained out and other amount of water is added to cause to cover the material left and heated again at 80° C for another 24 hours. This second water and extracted material is drained out again and both amounts joined together. This mother extract can be used alone alone for hair regrowth and skin and mucosas healing as bathing solution. Also the whole amount obtained is placed in a -vaccum dehydrator and concentrated at low temperature into a 20° Boumé concentrate. This concentrate can be added in the proportion of 1 to 5% into a mixture of water 60% and alcohol 96° at 40% to make a hair lotion, in 7 days variable amounts of hair regrowth will be observed. This regrowth will increase with persistan use of the lotion.

EXAMPLE 2:

The concentrate described above in EXAMPLE 1 can be lyophiliced and in 1% sutable pharmaceutical or cosmetic mixture in the form of cream or ointment can be used to heal sunburns, smal burns, first and second degree burns, abrations, excoriations and sores.

EXAMPLE 3:

By mixing one gram of the lyophilized powder to 40 grams of pure coconut oil plus 20 grams of animal pure fat and 39 grams of beeswax an excelent curative oinment is prepared for treating and curing skin sores and ulcers.

EXAMPLE 4:

By grinding finaly the dry cortex of the plant Mimosa Tuitenuiflora Leguminosae of one year of age or older and roasting this powder to slight -brown condition and applied every 24 hours on burns and ulcers of the skin and mucosas by powdering or insuflation in capsules for the stomach and in enemas diluted in water for the intestins, this lesions will happen to heal up in about one week.

It will be appreciated from the above description and the following claims -that various modifications may be maid to the invention without departing -from the scope thereof. It is intended that the various modifications will be within the scope of the present invention.

Claims

1- A process for producing a composition comprisinf the following steps:

(a)-extracting the soluble components of the cortex, small branches andleaves of the one year old or older plant Mimosa Tuitenuiflora Leguminosae from the Southeast region of MEXICO by means of water heated up to 80° C during at least 24 hours.

(b)-separating this extracts from the said cortex, small branches or leaves.

(c)-filtering the said extracts to obtain a clean preparation.

(d)-concentraiting the said extracts by means of vaccum dehydration and low heat application.

(e)-lyophilizing the said extract in(D) for further use to be kept as powder form for posterior use in different compositions.

2- A process for producing a composition comprising the following steps:

(a)extracting the soluble components of the cortex, small branches and leaves of the one year old or older plant Mimosa Tuitenuiflora Leguminosae from the Southeast part of MEXICO by means of alcoholes, etheres, acetones, glycerols, glycoles, petroleum derivatives solvents, mineral animal and vedgetable oils and fats, chromatographic procedures by simply grinding the parts of the plant said finely and inmersing them in the said substances and heating at no more that 80° C during enough period of time to extract the components and active ingridients.

(b)- filtering the said extracts to obtain clean and pure preparations.

(c) combining the extracts containing the curative active ingridients from the said plant Mimosa Tuitenuiflora Leguminosae with a pharmacological acceptable vehicule or ingridients or dilutants for applications on the scalp, skin and mucosas.

3- A method of causing to heal of bruises, excoriations, burns and ulcers either from the skin and mucosas through the application of the active ingridients extracted by the different procedures described above in Claims 1 and 2 mixed in any Pharmacological or cosmetic acceptable vehicule or diluent.

4- A method of causing dandruff from the scalp to desappear by the use of the active ingredients extracted by the different procedures described above in claims 1 and 2 mixed in any pharmacological or cosmetic acceptable vehicule or ingridient.

5- A method of promoting scalp hair regeneration and regrowth through the application of the active ingredients extracted by the different procedures described above in Claims 1 and 2 mixed in any Pharmacological or cosmetic acceptable vehicule or diluent.

6- A method of causing to heal of burns of first and second degrees and skin and mucosa wounds and ulcers through the application of the ground and sligh tly roasted cortex in the form of fine powder from the tree Mimosa Tuitenuiflora Leguminosae by powdering or insuflating it on said lesions.

7- The method of using the active ingridients from the cortex of the tree Mimosa Tuitenuiflora Leguminosae incorporated into gums natural or sintetic chewable for treating and healing teeth and mouth canker sores bychewing -this composition one or twice dayly for several days which also will heal teeth cavities.

8- The method of using the active ingredients of the tree Mimosa Tuitenuiflora Leguminosae in form of dilution, extracts, powders or mixtures with sutable and convinient Pharmacological ingridients and containers such as capsules of gelatin or sounds to put or introduce in contact with stomach and intestinal ulcers and lesions to cause them to heal in a short time.

9- The method of using the active extracts and components of the tree Mimosa Tuitenuiflora Leguminosa in a sutable Pharmacological compounds that upon oral administration causes migrane headhaches to subside in a short period of time.

10- The method of using the active ingredients and components of the tree Mimosa Tuitenuiflora Leguminosa in a sutable Pharmacological compound that upon oral or parenteral administration causes rehumatic conditions of muscles, tendosn, sinovial membranes and joints to subside and heal in a period no longer than one month.